# FASCICULE DE BREVET EUROPEEN

(11) **EP 1 062 908 B1**
(45) Date de publication et mention de la délivrance du brevet: **06.10.2004**
(21) Numéro de dépôt: 00401806.5
(22) Date de dépôt: 23.06.2000
(51) Int. Cl.: A61B 5/0408, A61B 5/0428, A61B 5/0432

(54) **Appareil de type Holter pour l'enregistrement de signaux physiologiques d'activité cardiaque.**
Gerät der Bauart Holter zur Aufzeichnung von physiologischen Signalen der Herztätigkeit
Holter type apparatus for recording physiological signals of cardiac activity

(30) Priorité: 23.06.1999 FR 9908028
(43) Date de publication de la demande: 27.12.2000
(73) Titulaire: ELA MEDICAL (Société anonyme), 92541 Montrouge (FR)
(72) Inventeur: Faisandier, Yves, 75116 Paris (FR)
(74) Mandataire: Dupuis-Latour, Dominique

(56) Documents cités:
- EP-A- 0 284 943
- FR-A- 2 554 704
- FR-A- 2 748 928
- US-A- 3 943 918
- US-A- 4 181 134

## Description

L'invention concerne les appareils de type "Holter" pour l'enregistrement de signaux physiologiques d'activité cardiaque recueillis par des électrodes externes appliquées à un patient.

L'invention vise plus particulièrement les appareils portatifs de ce type destinés à l'enregistrement ambulatoire en continu et sur une longue période des signaux d'activité cardiaque.

Cette technique utilise traditionnellement un boîtier enregistreur analogique ou numérique comportant une mémoire (cassette magnétique ou mémoire numérique statique) et des piles, sur lesquels se connectent des câbles se fixant sur les électrodes. Le boîtier est porté en bandoulière ou à la ceinture par le patient.

Un appareil selon la préambule de la revendication 1 est connu du document FR-A-2554704.

L'inconvénient majeur de ces appareils provient de l'inconfort du patient lié aux câbles : lorsqu'il se change de vêtements, qu'il prend sa douche ou son bain, les câbles viennent se prendre dans les vêtements, l'appareil ne peut rester en place et risque de tomber par terre, etc. De même, lorsqu'il dort, le patient doit poser l'appareil à proximité de lui, et risque ainsi de l'écraser ou d'arracher les câbles en se levant. Enfin certains vêtements, en particulier féminins, sont mal adaptés au passage de câbles entre le thorax et l'enregistreur porté en bandoulière.

L'un des buts de l'invention est de remédier à ces inconvénients, en proposant une nouvelle structure d'appareil Holter qui soit particulièrement compacte et minimise l'inconfort du patient.

L'idée de base de l'invention est de remplacer les électrodes et leurs fils par un ensemble unitaire et monobloc ou "socle" incorporant les électrodes et leurs liaisons électriques, et sur lequel sera directement monté le boîtier enregistreur. Ce dernier, au lieu d'être fixé à la ceinture ou porté en bandoulière, sera soutenu par le socle, l'ensemble socle-boîtier constituant donc un ensemble à la fois dépourvu de fils et autoporté par les électrodes.

Plus précisément, l'appareil de l'invention comporter un socle formé d'une feuille souple portant des électrodes et des conducteurs de liaison, ce socle comportant une région centrale recevant un boîtier enregistreur et pourvue de plages de contact formant les terminaisons proximales des conducteurs de liaison respectifs, et le boîtier enregistreur est pourvu de moyens de fixation au socle dans la région centrale de celui-ci, et de contacts d'électrode dont la configuration est homologue de celle des plages de contact du socle.

Selon diverses caractéristiques subsidiaires avantageuses :
- la région centrale du socle, ou une ceinture thoracique, ou encore un pendentif, forme support pour le boîtier ;
- le socle porte également une pile d'alimentation du boîtier enregistreur ;
- le socle est formé d'une feuille de circuit imprimé souple portant un motif conducteur gravé formant lesdites électrodes, lesdits conducteurs et lesdites plages de contact ;
- le boîtier enregistreur est formé d'une base et d'un couvercle articulés mutuellement le long d'un côté commun de manière à venir, en position fermée, pincer le socle dans la région centrale de celui-ci ; avantageusement, le boîtier enregistreur comporte également un joint périphérique d'étanchéité entourant lesdits contacts d'électrode et, le cas échéant, le(s) contact(s) de pile d'alimentation ;
- le boîtier et le socle comportent des moyens respectifs coopérants de centrage mutuel et de maintien, par exemple des plots coopérant avec des trous homologues.

On va maintenant décrire un exemple de mise en oeuvre de l'invention, en référence aux dessins annexés.

La figure 1 montre, en plan, le socle avec les électrodes.

La figure 2 montre ce socle mis en place sur le thorax du patient, avant fixation du boîtier.

La figure 3 est une vue schématique perspective du boîtier enregistreur, en position ouverte, avec ses différents éléments de liaison électrique et mécanique au socle.

La figure 4 montre, en coupe, le détail d'un contact de liaison du boîtier.

La figure 5 montre schématiquement, en perspective, le boîtier enregistreur refermé sur le socle.

Comme on l'a expliqué plus haut, l'appareil de l'invention est un appareil Holter autoporté par les électrodes, constitué de deux éléments distincts, à savoir un ensemble monobloc ou "socle" comportant les électrodes, d'une part, et le boîtier enregistreur qui se fixe sur le socle, d'autre part.

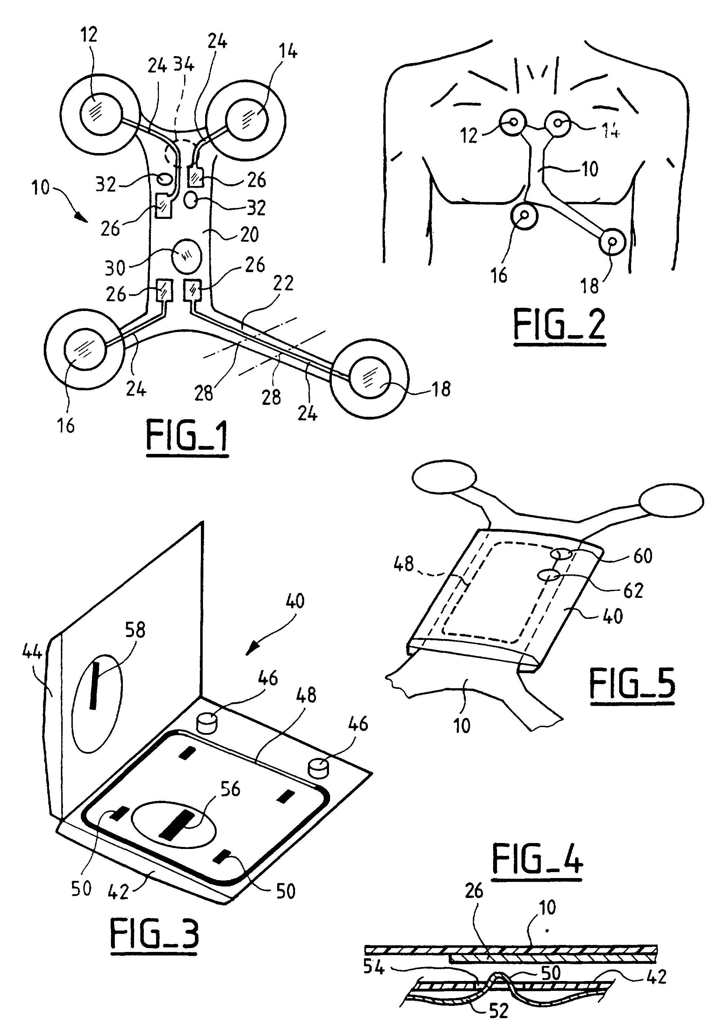

Sur les fig. 1 et 2, la référence 10 désigne de façon générale le socle, qui est un élément monobloc portant les électrodes, ici au nombre de quatre, à savoir deux électrodes supérieures 12, 14 et deux électrodes inférieures 16, 18. Ces électrodes sont d'un type en lui-même connu et sont fixées sur le thorax (fig. 2) soit par adhésion soit par dépression au moyen de ventouses.

Le socle 10 est avantageusement réalisé sous la forme d'un circuit imprimé souple, sur lequel sont dessinées et découpées les électrodes. La réalisation de celles-ci consiste à déposer une fine couche Ag-AgCl sur le métal du circuit imprimé (pour assurer un interfaçage optimal au contact de la peau). Un anneau d'adhésif et un gel de contact ou adhésif conducteur autour de l'électrode assureront les qualités requises pour le recueil du signal électrocardiographique.

En variante, il est également possible de prévoir des électrodes et/ou des conducteurs de liaison rapportés sur le circuit souple, l'ensemble devant toutefois constituer un élément monobloc unitaire.

Il est également possible de prévoir des électrodes dites "actives", c'est à dire dans lesquelles la tête d'amplificateur (amplificateur suiveur) est intégrée à l'électrode ; cette technique procure directement en sortie de l'électrode un niveau de signal élevé, permettant notamment de travailler sans avoir besoin d'appliquer un gel conducteur au contact de la peau.

On remarquera cependant qu'en tout état de cause, du fait que les conducteurs reliant chacune des électrodes à sa plage de contact respective, donc à l'enregistreur, ont une longueur réduite au minimum, beaucoup plus courte qu'avec un appareil Holter classique utilisant des fils volants, le niveau de bruit sera en toute hypothèse inférieur au niveau de bruit des appareils Holter classiques, même avec de simples électrodes passives.

Le socle 10 comporte une région centrale allongée 20 dont les deux extrémités sont prolongées par des bras tels que 22 s'étendant jusqu'aux électrodes et portant des pistes de liaison formant conducteurs 24 de liaison aux électrodes respectives. Chaque conducteur 24 est électriquement relié à l'électrode à son extrémité distale et se termine par une plage de contact 26 à son extrémité proximale située sur la région centrale 20, de manière que les liaisons aux différentes électrodes soient toutes regroupées sur cette même région centrale 20.

La position des électrodes, c'est à dire la configuration générale, la longueur et l'orientation des bras 22, doit de préférence correspondre aux standards de l'enregistrement Holter, avec une bonne séparation des axes des dérivations. La structure inextensible d'un circuit imprimé souple ne permettant pas de s'adapter spontanément à toutes les corpulences, ceci impose de prévoir pour les électrodes les plus éloignées soit un système avec des plis (tels que 28) ou des formes complexes, soient des modèles de plusieurs tailles.

De plus, la région centrale 20, qui servira à la fixation de l'enregistreur, doit permettre un maximum de confort, une excellente tenue et une distance minimum entre les électrodes et les plages de contact afin d'éviter au mieux les interférences.

Les conducteurs 24 entre les électrodes et les plages de contact sont isolés électriquement du patient à l'aide d'un vernis ou d'une couche d'isolant compatibles avec les contraintes de contact avec le corps.

A l'endroit des plages de contact 26, le cuivre du circuit imprimé est revêtu d'un dépôt de nickel ou d'une très fine couche d'or pour assurer une protection efficace contre l'oxydation.

Le socle 10 peut comporter une pile 30 d'alimentation du boîtier enregistreur qui sera placé sur ce socle, ou bien un condensateur de forte capacité (par exemple 1 farad). Il est également pourvu de trous 32 de positionnement mécanique du boîtier enregistreur, dont on expliquera le rôle plus bas.

Le socle 10 ainsi constitué est considéré comme un élément consommable, changé à chaque examen comme cela était fait jusqu'à présent d'une part pour le jeu d'électrodes et d'autre part pour les piles du boîtier enregistreur. Le socle pouvant incorporer la pile, le changement du socle revient à préparer, en une seule action, l'appareil pour un nouvel enregistrement, sans autre manipulation.

Le socle peut en outre être équipé d'un blindage le long des lignes de liaison aux électrodes, ainsi que d'une cinquième électrode de masse, référencée 34 sur la fig. 1, qui peut servir accessoirement d'attache pour le maintien du socle au thorax. En effet, il est conseillé de ne jamais tirer sur les fils d'une électrode, ceci provoquant un mouvement entre les surfaces métalliques et la peau pouvant créer une tension parasitant l'enregistrement de l'ECG. En plaçant, comme illustré, l'électrode de masse 34 au point de jonction des deux électrodes supérieures 12, 14, celle-ci peut alors supporter le poids de l'appareil, et les liaisons aux électrodes de recueil peuvent rester mécaniquement flottantes. En variante, il est possible d'assurer cette fonction mécanique avec un simple adhésif passif, sans électrode de masse, si cette dernière n'est pas nécessaire pour obtenir de bonnes performances électriques.

La fig. 3 montre le boîtier enregistreur 40, destiné à coopérer avec le socle 10. Ce boîtier, qui doit être monté sur le socle 10 et soutenu par celui-ci, comporte une pince-connecteur venant soit entourer le socle (comme dans l'exemple illustré), soit s'accrocher dessus.

Dans le cas d'un socle en circuit imprimé souple, le boîtier 40 peut notamment être réalisé sous la forme illustrée fig. 3, avec une partie inférieure ou base 42 sur laquelle vient se rabattre un couvercle articulé 44, l'ensemble venant, en position fermée, pincer le socle 10 dans sa partie centrale 20, comme illustré sur la fig. 5.

Le positionnement précis de la base 42 par rapport au socle 10 est assuré par des plots de centrage et de maintien 46 ou organes analogues (par exemple des encoches placées à la périphérie du boîtier 40) venant coopérer avec les trous 32 du socle 10.

Il est prévu par ailleurs un joint d'étanchéité 48 entourant la zone comportant les contacts. Lorsque le boîtier est fermé, le joint périphérique 48 vient s'écraser sur le circuit imprimé du socle 10 en empêchant toute introduction de liquide dans la zone de contact socle/boîtier ; un ou plusieurs autres joints peuvent être éventuellement placés sur le couvercle 44 pour assurer l'étanchéité d'un éventuel contact de pile supérieur 58 (voir plus bas).

La base 42 du boîtier 40 est pourvue de contacts 50 homologues des plages de contact 26 du socle. Ces contacts sont par exemple réalisés de la manière illustrée fig. 4, au moyen d'une lame-ressort élastiquement déformable 52 émergeant d'un orifice 54 du boîtier de la base 42, de manière à venir en appui élastique contre la plage de contact 26 du socle 10. De préférence, les contacts 50 sont tous disposés sur la base 42 du boîtier enregistreur de façon à n'utiliser, pour la fabrication du socle 10, qu'un circuit imprimé simple face. La base comporte également une électrode de contact 56 avec l'un des pôles de la pile 30 ; si cette pile est une pile bouton traversante (c'est à dire dont les pôles respectifs sont situés au recto et au verso du circuit imprimé du socle 10), il est nécessaire de prévoir sur le couvercle 44 un contact 58 pour la liaison avec cette pile ; dans le cas d'une pile plate, il est possible et préférable de ramener tous les contacts de pile du même côté, donc de les regrouper sur la base 42 avec les autres contacts.

Le boîtier enregistreur 40 comporte une électronique de recueil et de traitement des signaux ECG, qui est en elle-même classique et ne sera pas décrite plus en détail. Les circuits électroniques sont par exemple comparables à ceux du modèle *Syneflash* de la société ELA Médical.

L'ensemble des circuits électroniques est avantageusement regroupé dans la partie de base 42 du boîtier enregistreur 40, si l'on désire minimiser les liaisons entre la base 42 et le couvercle 44 ; ainsi, si tous les contacts sont regroupés sur la base, le couvercle peut être simplement constitué d'une pièce en matière plastique.

Lors de la fermeture, l'enregistreur se met de lui-même sous tension, soit grâce au contact de pile, soit par un interrupteur mécanique ou électronique supplémentaire ; un verrou mécanique bloque la fermeture de l'appareil.

Une fois le boîtier fermé, celui-ci laisse apparaître, comme illustré fig. 5, un témoin lumineux et/ou sonore 60 permettant d'en vérifier le bon fonctionnement, et une fenêtre 62 d'un port de transmission infrarouge de données, par exemple du type *IrdA,* pour l'échange bidirectionnel de données et de commandes entre le processeur de l'enregistreur Holter et le système associé de programmation et d'exploitation des données.

Diverses variantes de réalisation peuvent être envisagées.

Ainsi, au lieu de prévoir la pile 30 sur le socle 10, il est possible de placer celle-ci à l'intérieur du boîtier enregistreur 40. Si l'électronique a une consommation extrêmement réduite, une simple pile "bouton" de quelques grammes peut alors alimenter le boîtier pendant plusieurs mois, au lieu de la changer à chaque examen lorsqu'elle est intégrée au socle. Il est alors avantageux de prévoir un dispositif de contrôle de la tension des piles afin de prémunir l'utilisateur d'une pile trop usée.

Diverses variantes de réalisation sont bien entendu envisageables. Ainsi, dans l'exemple décrit plus haut, le boîtier enregistreur 40 est entièrement soutenu par le socle 10 qui fait donc fonction, entre autres, de moyen support du boîtier. Cette caractéristique n'est cependant pas indispensable, et il est possible de prévoir que le boîtier ne soit plus porté par le socle, mais porté par une ceinture thoracique (à la manière des cardiofréquencemètres pour sportifs) ou soutenu par un collier (à la manière d'un pendentif). Du fait des moindres sollicitations mécaniques exercées à l'interface socle/peau, il est alors possible de s'affranchir de l'adhésif de maintien des électrodes, celles-ci étant alors fixées par exemple par un système à dépression (l'énergie de dépression pouvant provenir du boîtier). L'absence d'adhésif est notamment souhaitable lorsque le dispositif de l'invention est utilisé par des sportifs, ou encore des patients sujets à une forte transpiration ou à des allergies cutanées.

## Revendications

1. Un appareil Holter pour l'enregistrement de signaux physiologiques d'activité cardiaque recueillis par des électrodes externes appliquées à un patient, cet appareil comportant un jeu d'électrodes (12,14,16,18), un boîtier enregistreur (40) et des conducteurs de liaison (24) des électrodes au boîtier,
**caractérisé par** un socle (10) formé d'une feuille souple portant les électrodes (12, 14, 16, 18) et les conducteurs de liaison (24), ce socle comportant une région centrale (20) recevant le boîtier enregistreur (40) et pourvue de plages de contact (26) formant les terminaisons proximales des conducteurs de liaison respectifs,
et **en ce que** le boîtier enregistreur est pourvu de moyens de fixation (32,46) au socle dans la région centrale de celui-ci, et de contacts d'électrode (50) dont la configuration est homologue de celle des plages de contact (26) du socle.

2. L'appareil de la revendication 1, dans lequel la région centrale du socle forme support pour le boîtier.

3. L'appareil de la revendication 1, dans lequel une ceinture thoracique forme support pour le boîtier.

4. L'appareil de la revendication 1, dans lequel un pendentif forme support pour le boîtier.

5. L'appareil de la revendication 1, dans lequel le socle porte également une pile (30) d'alimentation du boîtier enregistreur.

6. L'appareil de la revendication 1, dans lequel le socle est formé d'une feuille de circuit imprimé souple portant un motif conducteur gravé formant lesdites électrodes, lesdits conducteurs et lesdites plages de contact.

7. L'appareil de la revendication 1, dans lequel le boîtier enregistreur (40) est formé d'une base (42) et d'un couvercle (44) articulés mutuellement le long d'un côté commun de manière à venir, en position fermée, pincer le socle (10) dans la région centrale (20) de celui-ci.

8. L'appareil de la revendication 7, dans lequel le boîtier enregistreur comporte un joint périphérique d'étanchéité (48) entourant lesdits contacts d'électrode (50) et, le cas échéant, le(s) contact(s) de pile d'alimentation (56).

9. L'appareil de la revendication 1, dans lequel le boîtier et le socle comportent des moyens respectifs coopérants (32, 46) de centrage mutuel et de maintien.

10. L'appareil de la revendication 9, dans lequel les moyens respectifs de centrage mutuel et de maintien comprennent des plots (46) coopérant avec des trous (32) homologues.

11. L'appareil de la revendication 1, dans lequel le boîtier enregistreur est pourvu de moyens de transmission de données, notamment de transmission infrarouge, entre un processeur de l'appareil Holter et un système associé de programmation et d'exploitation de ces données.

## Patentansprüche

1. Holter-Gerät zur Aufzeichnung von physiologischen Signalen der Herztätigkeit, die durch externe Elektroden empfangen werden, die einem Patienten angelegt wurden, wobei dieses Gerät einen Satz von Elektroden (12, 14, 16, 18) aufweist, einen Aufzeichnungskasten (40) und Leiter zur Verbindung (24) der Elektroden mit dem Gehäuse,
**gekennzeichnet durch** einen Sockel (10), gebildet aus einer geschmeidigen Folie, welche die Elektroden (12, 14, 16, 18) und die Leiter zur Verbindung (24) trägt, wobei der Sockel einen zentralen Bereich (20) aufweist, welcher den Aufzeichnungskasten (40) aufnimmt und mit Kontaktbereichen (26) versehen ist, die proximale Enden der jeweiligen Leiter zur Verbindung bilden,
und **dadurch**, dass der Aufzeichnungskasten versehen ist mit Befestigungsmitteln (32, 46) am Sockel in dem zentralen Bereich desselben, und mit Elektrodenkontakten (50), deren Konfiguration homolog zur derjenigen der Kontaktbereiche (26) des Sockels ist.

2. Gerät gemäß Anspruch 1, in welchem der zentrale Bereich des Sockels eine Unterstützung für den Kasten bildet.

3. Gerät gemäß Anspruch 1, in welchem ein Brustgurt eine Unterstützung für den Kasten bildet.

4. Gerät gemäß Anspruch 1, in welchem ein Pendentif eine Unterstützung für den Kasten bildet.

5. Gerät gemäß Anspruch 1, in welchem der Sockel gleichfalls eine Batterie (30) aufweist zur Versorgung des Aufzeichnungskastens.

6. Gerät gemäß Anspruch 1, in welchem der Sockel gebildet wird aus einer geschmeidigen Folie mit aufgedrucktem Schaltkreis, welche ein eingraviertes Leitungsmuster trägt, welche die Elektroden, die Leiter und die Kontaktbereiche bildet.

7. Gerät gemäß Anspruch 1, in welchem der Aufzeichnungskasten (40) gebildet wird aus einer Basis (42) und einer Abdeckung (44), wechselseitig ausgebildet entlang einer gemeinsamen Seite, um, in geschlossener Position, den Sockel (10) in dem zentralen Bereich (20) desselben einzuklemmen.

8. Gerät gemäß Anspruch 7, in welchem der Aufzeichnungskasten eine Dichtung (48) aufweist, welche die Elektrodenkontakte (50) umgibt und, gegebenenfalls, den/die Kontakt(e) der Versorgungsbatterie (56).

9. Gerät gemäß Anspruch 1, in welchem der Kasten und der Sockel jeweils zusammenwirkende Mittel (32, 46) der wechselseitigen Zentrierung und des Haltens aufweisen.

10. Gerät gemäß Anspruch 9, in welchem die jeweiligen Mittel der wechselseitigen Zentrierung und des Haltens Stifte (46) aufweisen, welche mit homologen Löchern (32) zusammenwirken.

11. Gerät gemäß Anspruch 1, in welchem der Aufzeichnungskasten versehen ist mit Mitteln zur Übertragung von Daten, insbesondere der Infrarot-Übertragung, zwischen einem Prozessor des Holter-Geräts und einem zugehörigen System zur Programmierung und Verwertung dieser Daten.

## Claims

1. A Holter apparatus for recording cardiac activity physiological signals collected by external electrodes applied to a patient, said apparatus comprising a set of electrodes (12, 14, 16, 18), a recording case (40), and conductors (24) for connecting the electrodes to the case,
**characterised by** a base (10) formed of a flexible sheet carrying the electrodes (12, 14, 16, 18) and the connecting conductors (24), said base comprising a central area (20) for receiving the recording case (40) and provided with contact pads (26) forming the proximal terminations of the respective connecting conductors,
and in that the recording case further comprises means (32, 46) for securing it to the base in the central area of the latter, and electrode contacts (50) the arrangement of which matches the arrangement of the contact pads (26) of the base.

2. The apparatus of claim 1, wherein the central area of the base forms a support for the case.

3. The apparatus of claim 1, wherein a thoracic belt forms a support for the case.

4. The apparatus of claim 1, wherein a hanging strap forms a support for the case.

5. The apparatus of claim 1, wherein the base further supports a battery (30) for supplying power to the recording case.

6. The apparatus of claim 1, wherein the base is made from a sheet of flexible printed circuit supporting an etched conductive pattern forming said electrodes, said conductors, and said contact pads.

7. The apparatus of claim 1, wherein the recording case (40) is made from a base (42) and a cover (44) mutually hinged along a common side in order to grip, in a closed position, the base (10) in the central area (20) of the latter.

8. The apparatus of claim 7, wherein the recording case includes a peripheral seal (48) disposed around said electrode contacts (50) and, if any, the power battery contact(s) (56).

9. The apparatus of claim 1, wherein the case and the base include respective cooperating means (32, 46) for mutual centering and support.

10. The apparatus of claim 9, wherein the respective means for mutual centering and support comprise studs (46) cooperating with mating holes (32).

11. The apparatus of claim 1, wherein the recording case is provided with means for data transmission, in particular infrared transmission, between a processor of the Holter apparatus and an associated system for programming and processing said data.
